# EUROPÄISCHE PATENTANMELDUNG

(11) **EP 1 074 838 A2**
(43) Veröffentlichungstag der Anmeldung: **07.02.2001**
(21) Anmeldenummer: 00110049.4
(22) Anmeldetag: 12.05.2000
(51) Int. Cl.: G01N 33/24, G01N 33/00, G01N 33/497

(54) **Vorrichtung zur Untersuchung des Verhaltens von Umweltchemikalien unter natürlichen Bedingungen**

(30) Priorität: 27.07.1999 DE 19935133
(71) Anmelder: GSF-Forschungszentrum für Umwelt und Gesundheit GmbH, 85764 Neuherberg (DE)
(72) Erfinder: Schroll, Reiner, Dr., 86551 Aichach (DE)
(74) Vertreter: Maiwald, Walter, Dr. Dipl.-Chem.

(57) **Zusammenfassung**

Die Erfindung betrifft eine Vorrichtung zur Untersuchung des Verhaltens und des Abbaus von Umweltchemikalien im geschlossenen System, wobei die Vorrichtung mindestens ein im wesentlichen mit Boden gefülltes Behältnis, den Lysimeter, und eine oberhalb der Bodenoberfläche angeordnete Volatilitätskammer umfaßt, wobei die Volatitätskammer die flüchtigen Verbindungen aus einem definierten Bodenteilbereich erfaßt. Vorzugsweise umfaßt die Vorrichtung zusätzlich eine Pflanzenkammer, die flüchtige Verbindungen von einer ausgewählten Pflanze aus dem Pflanzenbestand erfaßt. Das erfindungsgemäße Testsystem (Lysimeteranlage) ermöglicht die Bestimmung der Verlagerung, des Transports, der Auswaschung, der Umwandlung (Metabolisierung), der Minerialisierung und der Verflüchtigung von Chemikalien unter natürlichen Klimabedingungen sowohl ohne den Einfluß von Pflanzen als auch unter dem Einfluß von Pflanzen in der im Lysimeter enthaltenen Bodensäule sowie die Mineralisierung und Verflüchtigung von Chemikalien unter natürlichen Klimabedingungen von Pflanzenoberflächen. Die Erfindung betrifft auch ein Verfahren zur Testung von Chemikalien in Böden unter Verwendung der erfindungsgemäßen Vorrichtung.

## Beschreibung

Boden gefülltes Behältnis (im folgenden auch Lysimeter genannt) und eine oberhalb der Bodenoberfläche angeordnete Volatilitätskammer umfaßt. In einer bevorzugten Ausführungsform umfaßt die Vorrichtung zusätzlich eine oberhalb der Bodenoberfläche angeordnete Pflanzenkammer.

Das erfindungsgemäße Testsystem, im folgenden auch Lysimeteranlage genannt, ermöglicht die Bestimmung der Verlagerung, des Transports, der Auswaschung, der Umwandlung (Metabolisierung), der Minerialisierung und der Verflüchtigung von Chemikalien unter natürlichen Klimabedingungen sowohl ohne den Einfluß von Pflanzen als auch unter dem Einfluß von Pflanzen in der im Lysimeter enthaltenen Bodensäule. Die Erfindung betrifft auch ein Verfahren zur Testung von Chemikalien in Böden unter Verwendung der erfindungsgemäßen Vorrichtung.

Im Gegensatz zu bekannten Testsystemen ermöglicht die erfindungsgemäße Lysimeteranlage die Untersuchung von Umweltchemikalien im Hinblick auf ihre Mineralisierung und/oder Verflüchtigung getrennt nach Pflanzen- und Bodenoberflächen. Bekannte Systeme bieten diese Möglichkeit nicht. Z.B. werden in den von der Biologischen Bundesanstalt für Land- und Forstwissenschaft, Braunschweig, vorgeschriebenen Versuchen zur Zulassung von Pflanzenschutzmitteln jeweils lediglich Einzelprozesse wie Verlagerung, Mineralisierung und Volatilisierung untersucht. Mit Hilfe der erfindungsgemäßen Lysimeteranlage können jedoch in einem Lysimeterversuch auch Informationen über die Mineralisierung und die Volatilität von Umwelt- und Agrochemikalien unter natürlichen Bedingungen erhalten werden. Dies kann aufwendige zusätzliche Laborversuche überflüssig machen. Zudem werden die zusätzlichen Ergebnisse unter natürlichen Klimabedingungen und nicht unter künstlichen Laborbedingungen gewonnen, was die Aussagekraft der Ergebnisse deutlich erhöht.

Die vorliegende Erfindung soll die kontinuierliche Erfassung des Verhaltens von Umweltchemikalien von Boden- und/oder Pflanzenoberflächen ermöglichen und dadurch die beschriebenen Nachteile des Standes der Technik vermeiden helfen. Es ist eine wesentliche Aufgabe der Erfindung, ein Lysimetersystem zur Verfügung zu stellen, mit dem das Verhalten von Umweltchemikalien im System Boden/Luft, gegebenenfalls im System Boden/Pflanze/Luft, untersucht werden kann. Hierbei soll es insbesondere möglich sein, die Verlagerung, die Umwandlung bis hin zur Mineralisierung, die Auswaschung und die Verflüchtigung von Umweltchemikalien sowohl ohne den Einfluß von Pflanzen, als auch unter dem Einfluß von Pflanzen in Bodensäulen zu untersuchen. In einer bevorzugten Ausführungsform handelt es sich um radioaktiv markierte, vorzugsweise ¹⁴C-markierte, Pflanzenschutzmittel bzw. andere Umweltchemikalien.

Diese und weitere Aufgaben, die sich aus der nachfolgenden Beschreibung ergeben, werden durch die vorliegende Erfindung gelöst, bei der es sich im Gegensatz zu bekannten Lysimeteranlagen um ein sogenanntes geschlossenes Testsystem handelt, das eine kontinuierliche Messung der gewünschten Parameter unter natürlichen Klimabedingungen erlaubt.

Die erfindungsgemäße Lysimeteranlage umfaßt mindestens ein im wesentlichen mit Boden gefülltes Behältnis und eine oberhalb der Bodenoberfläche angeordnete Volatilitätskammer, die die flüchtigen Bestandteile aus einem definierten Bodenteilbereich erfaßt. Bevorzugt umfaßt die erfindungsgemäße Lysimeteranlage weiter eine Pflanzenkammer, die die flüchtigen Bestandteile von ausgewählten Pflanzenoberflächen erfaßt.

Mit Hilfe der Isotopentracertechnik, insbesondere der ¹⁴C-Markierung, in Kombination mit konventionellen analytischen Methoden ermöglicht das erfindungsgemäße System eine gezielte Untersuchung der Aufnahme von Pflanzenschutzmitteln durch Pflanzen und deren Abgabe über oberirdische Pflanzentele an die Atmosphäre sowie zum Langzeitverhalten von Rückständen im System Boden/Pflanze/Luft. Besondere Berücksichtigung finden dabei Detailprozesse wie Abbau und Metabolisierung, Sorption, Verlagerung im Boden, Bildung und Bioverfügbarkeit von gebundenen Rückständen sowie insbesondere gasförmige Verluste durch Mineralisierung und Verflüchtigung. Des weiteren können sämtliche Rückstände der Umweltchemikalien, die von den Pflanzen über die Wurzeln aufgenommen und über die Blätter an die Atmosphäre abgegeben werden, kontinuierlich gemessen werden. Im Gegensatz zu allen bekannten Testsystemen ist die erfindungsgemäße Lysimeteranlage als "geschlossenes System" konzipiert (von den Teilflächen der Probenahme wird auf die gesamte Lysimeterfläche extrapoliert), was die Aufstellung einer vollständigen Massenbilanz für die markierte Umweltchemikalie, die auf der Bodenoberfläche bzw. auf den Pflanzenbestand appliziert wird, möglich macht.

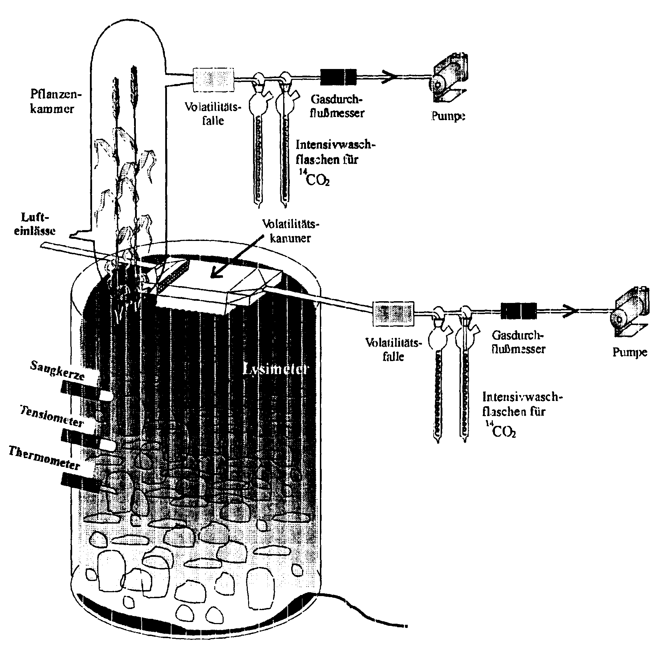

Die erfindungsgemäße Testanlage wird im folgenden unter Bezugnahme auf die beigefügte Abbildung 1 beschrieben, die eine bevorzugte Ausführungsform der Erfindung zeigt.

Das Zentrum der Lysimeteranlage bildet der Lysimeter, d.h. ein im wesentlichen mit Boden gefülltes Behältnis. Vorzugsweise handelt es sich um ein zylinderförmiges Gefäß aus Metall oder einem anderen geeigneten Material, welches inert ist, also die Massenbilanz nicht beeinflußt. Vorzugsweise hat das Behältnis einen Durchmesser von ca. 110 cm und eine Höhe von ca. 100 - 200 cm. Am unteren Ende ist das Behältnis durch einen separaten Behälter, der mit Sand unterschiedlicher Körnung gefüllt ist, abgeschlossen. Der Lysimeter selbst ist mit Boden gefüllt.

Nach der Applikation der zu untersuchenden Umweltchemikalie auf die Bodenoberfläche bzw. auf den Pflanzenbestand wird möglichst umgehend eine sogenannte Volatilitätskammer auf einen Teil der Bodenoberfläche gepreßt und an ein weiter unten beschriebenes "Fallensystem" angeschlossen. Anschließend wird, sofern Pflanzen in die Untersuchung einbezogen werden sollen, eine repräsentative Pflanze aus dem Pflanzenbestand ausgewählt und in eine sogenannte, ebenfalls weiter unten beschriebene "Pflanzenkammer" präpariert. Diese Kammer wird an ein zweites "Fallensystem" angeschlossen.

Die Volatilitätskammer dient dazu, aus einem genau definierten Bodenbereich flüchtige markierte, insbesondere ¹⁴C-markierte, Verbindungen, d.h. vor allem flüchtige organische ¹⁴C-markierte Organika, sowie ¹⁴CO₂, getrennt voneinander zu erfassen. Bevorzugt hat der eigentliche Beprobungsbereich der Volatilitätskammer eine Größe von 8 x 20 cm (Breite x Länge). Dem Beprobungsbereich vorgelagert ist eine Zone (8 x 4 cm; Breite x Länge), die zur Erzeugung einer laminaren Luftströmung dient. Eine zweite Zone (8 x 4 cm; Breite x Länge), die zur Zusammenführung der Luftströmung in der Volatilitätskammer dient, ist der eigentlichen Beprobungsfläche nachgelagert. Um den Beprobungsbereich eindeutig abzugrenzen, ist am unteren Ende der Kammer ein Stechzylinder mit den Maßen 8 x 20 x 5 cm (Breite x Länge x Tiefe) angebracht. Die Kammer wird bis zum oberen Rand des Stechzylinders in den Boden gedrückt, damit gewährleistet ist, daß nur dieser Bodenbereich beprobt werden kann. Die Luft, die mittels einer Pumpe durch die Kammer gesaugt wird, wird anschließend in einem Fallensystem ausgewaschen. Die durch die Kammer geleitete Luftmenge beträgt bevorzugt ungefähr 1200 l/h.

Das erfindungsgemäße Fallensystem besteht in erster Linie aus einem oder mehreren Polyurethanschaumfiltern und einer oder mehreren sogenannten Intensivwaschflaschen. Dabei wird die durch die Volatilitätskammer gezogene Luft zunächst durch einen in Abbildung 1 als Volatilitätsfalle dargestellten PU-Schaumfillter und anschließend durch vorzugsweise mindestens zwei sogenannte Intensivwaschflaschen gesaugt. Anstelle des PU-Schaums kann jeder andere für volatile Substanzen geeignete Filter, beispielsweise eine Flüssigkeitsfalle, eingesetzt werden.

Die Intensivwaschflaschen sind mit einer reaktiven Flüssigkeit, vorzugsweise mit einer Mischung aus Ethanolamin und Diethylenglykolmonobutylether (z.B. im Verhältnis 3:8,5) gefüllt. Durch ein Glasrohr wird die zu filternde Luft an das untere Ende der Intensivwaschflasche gesaugt und durch eine Glasfritte zwangsweise wieder nach oben geführt. Durch die Form der Glasteile und den durch eine Pumpe erzwungenen Luftstrom entstehen kleine Luftblasen, die durch die Flüssigkeit nach oben perlen. Dadurch wird die Luft intensiv "ausgewaschen". Derartige "Fallensysteme" sind beispielsweise beschrieben in Schroll R. und Scheunert I. (1992), Chemosphere, Vol. 24, No. 1, Seiten 97 - 108. In einer bevorzugten Ausführungsform befindet sich am oberen Ende der Intensivwaschflaschen eine "Kühlzone", die mit Hilfe eines Kryostaten gekühlt wird und an der Flüssigkeitsmoleküle, die mit dem Luftstrom mitgerissen werden, kondensieren und wieder in die eigentliche Intensivwaschflasche zurücktropfen können. Da sich durch die Reaktion der Flüssigkeiten mit der Luft die Viskosität der Flüssigkeit stark erhöht, wird über der Glasfritte ein zweiter Ablaßhahn eingebaut, um das Entleeren der Intensivwaschflaschen zu erleichtern.

Mit Hilfe zweier verschiedener Gasdurchflußmesser wird der Luftstrom durch das gesamte System auf die entsprechenden Durchflußraten eingestellt. Pumpen hinter den Gasdurchflußmessern sorgen für den entsprechenden Luftfluß durch das System. Dabei wird, wie oben erwähnt, sowohl für die Volatilitätskammer, als auch für die Pflanzenkammer ein spezielles "Fallensystem" benötigt, um die verschiedenen flüchtigen markierten Verbindungen aus der Luft auswaschen zu können. Die Luft aus beiden Systemen wird getrennt voneinander durch jeweils einen Polyurethanschaumfilter - um die flüchtigen organischen Verbindungen zu adsorbieren - und jeweils zwei sogenannte Intensivwaschflaschen - um das ¹⁴CO₂ aus der Luft aufzufangen - gesaugt. Die gesamte Luftmenge (ca. 1200 l/h) aus den zwei verschiedenen Kammersystemen wird durch den Polyurethanschaum gefiltert. Diese gesamte Luftmenge kann jedoch nicht durch die Intensivwaschflaschen geführt werden, weshalb hier ein genau definierter Teiluftstrom von z.B. jeweils 200 l/h abgezweigt und über die sogenannten Intensivwaschflaschen gefiltert wird.

Die in Abbildung 1 gezeigte Pflanzenkammer wird wie folgt eingerichtet. Am unteren Ende eines ca. 20 cm langen Glasrohres ist mittig ein kleines Glasrohr mit Gewinde angebracht, das mittels einer Irisblende nahezu vollständig abgedichtet werden kann. Nach der Applikation der Testsubstanz wird durch die Irisblende eine ausgewählte Pflanze präpariert. Mittels der Irisblende kann die Pflanzenkammer gegen die Atmosphäre weitgehend abgeschlossen werden. Um völlig ausschließen zu können, daß trotz des Verschließens der Irisblende Luft aus dem bodennahen Bereich - und somit auch flüchtige ¹⁴C-markierte Verbindungen, die aus dem Boden emittieren - in die Pflanzenkammer gesogen werden könnte und somit auch die Versuchsergebnisse verfälschen würde, wird mittels einer Pumpe und einer Düse gefilterte Luft an die Irisblende gedrück, um auf diese Weise eine Art Windbarriere zu errichten.

Das beschriebene Glasrohr der Pflanzenkammer wird durch eine geeignete Halterung auf dem Lysimeterboden verankert. Ein zweites Glasrohr mit einer Länge von ca. 130 cm mit O-Ring schließt die Pflanzenkammer nach oben ab. Luft, die durch die Pflanzenkammer mittels einer Pumpe gezogen wird, wird in einem oben beschriebenen Fallensystem, das mit der Pflanzenkammer verbunden ist, ausgewaschen. Die durch die Kammer gezogene Luftmenge beträgt bevorzugt ungefähr 1200 l/h.

Nach der Applikation der Testsubstanz auf die Lysimeteroberfläche auf herkömmliche Weise werden die Testsysteme "Volatilitätskammer" und "Pflanzenkammer" möglichst umgehend nach der Applikation auf der Lysimeteroberfläche installiert. Die Volatilitätskammer wird dabei vorsichtig per Hand zwischen die Pflanzenreihen in den Boden gedrückt. Es ist darauf zu achten, daß keine Pflanze beschädigt wird. Anschließend wird die Kammer mit dem hierfür vorgesehenen Fallensystem verbunden.

Aus dem Pflanzenbestand wird eine repräsentative Pflanze ausgewählt und vorsichtig durch die Irisblende in die Pflanzenkammer präpariert. Auch hierbei muß streng darauf geachtet werden, daß die Pflanze nicht verletzt wird. Anschließend wird die Pflanzenkammer an das hierfür vorgesehene Fallensystem angeschlossen.

Für die kontinuierliche Messung werden die diversen Falleninhalte regelmäßig befüllt und entleert. Dabei werden die Proben aus den Intensivwaschflaschen zu gewünschter Zeit in einen Meßzylinder abgelassen und das Volumen bestimmt. Aliquots werden in einem geeigneten Szintillationscocktail (z.B. Hionic Fluor von Canberra Packard, Frankfurt) zur Messung gebracht.

Volatile ¹⁴C-Bestandteile werden mittels der oben erwähnten Polyurethan-Fallen aufgefangen. Die PU-Schwämme werden dann regelmäßig gewechselt und präpariert. Dabei werden für die Volatilitätsmessung die Schwämme aus den Gasfallen entfernt und mit Aceton oder anderen geeigneten Lösungsmitteln ausgewaschen und die Lösungsmittelextrakte gegebenenfalls anschließend am Rotationsverdampfer eingeengt. Zur Szintillationsmessung wird ein geeigneter Szintillationscocktail, beispielsweise Ultima Gold XR (erhältlich von der Firma Canberra Packard, Frankfurt), verwendet.

Für die Analyse des Verhaltens der zu untersuchenden Umweltchemikalie, insbesondere die Aufstellung einer Massenbilanz, können herkömmliche Methoden eingesetzt werden, wie sie beispielsweise bei Schroll R. und Scheunert I., Supra Schroll R. und Scheunert I. (1993) Chemosphere, Vol. 26, No. 9, Seiten 1631 - 1640, Schroll R. et al. (1994) Chemosphere, Vol. 28, No. 2, Seiten 297 - 303 und Gayler S. et al. (1995) Environ. Sci. & Pollut. Res., Vol. 2, No. 2, Seiten 98 - 103, beschrieben sind.

Zusätzlich können nach Beendigung des Versuchs die Versuchspflanzen aufgearbeitet werden, wobei die Aufarbeitung vorzugsweise getrennt nach unter- und oberirdischen Pflanzenteilen erfolgt. Die Blätter und Stengel werden geeigneterweise in flüssigen Stickstoff getaucht und in einer Mühle pulverisiert. Das Pflanzenpulver kann anschließend mit entsprechenden Lösungsmitteln extrahiert und der Extrakt gegebenenfalls nach Aufreinigung mittels Dünnschichtchromatographie, Gelpermeationschromatographie oder Gegenstromchromatographie, mittels einer HPLC analysiert werden.

Zusätzlich werden die Pflanzenkammern innen mit Lösungsmittel, beispielsweise Aceton oder Methanol sorgfältig gewaschen, das gesamte Waschlösungsmittel gesammelt und am Rotationsverdampfer eingeengt. Nach Aufnahme in einer geeigneten Menge an Lösungsmittel wird im Szintillationszähler der Gehalt an radioaktiv markiertem Kohlenstoff bestimmt. Eine Identifizierung der Rückstände kann z.B. mittels einer HPLC mit ¹⁴C-Detektor erfolgen.

Schließlich werden auch die Bodenproben einer Rückstandsanalytik unterzogen, wobei die Bodenproben Schicht für Schicht extrahiert werden, die Menge an extrahierbarer ¹⁴C-Radioaktivität im Szintillationszähler erfaßt wird und mit Hilfe chromatographischer Methoden (HPLC, GC, MS) die einzelnen Substanzen identifiziert werden. Nicht extrahierbare Rückstände werden mit Hilfe eines Verbrennungsautomaten (Sample Oxidier, z.B. Firma Canberra Packard, Frankfurt) quantifiziert. Auf diese Weise ermöglicht die komplette Rückstandsanalytik die Aufstellung einer vollständigen Massenbilanz.

Ergänzende Untersuchungen von Sickerwässern können ebenfalls nach konventionellen Methoden durchgeführt werden.

Im folgenden soll das erfindungsgemäße Verfahren mittels der erfindungsgemäßen Lysimeteranlage im Detail beschrieben werden:

Nach Applikation der Testsubstanz auf der Lysimeteroberfläche und Installieren der Volatilitätskammer und Pflanzenkammer auf der Lysimeteroberfläche werden die CO₂-Fallen dreimal wöchentlich entleert, d.h. die Proben aus den Intensivwaschflaschen werden entnommen und die Flaschen neu befüllt.

Die PU-Schwämme werden zwei- bis dreimal wöchentlich gewechselt. Darauf folgt die Präparation der ¹⁴CO₂-Proben und der Einbau der Schwämme in die Soxhlet-Extraktionsanlage (die möglichst über Nacht laufen sollte). Die Extrakte der PU-Schwämme werden falls erforderlich am Rotationsverdampfer eingeengt.

Im Detail erfolgt die ¹⁴CO₂-Probenentnahme und Präparation wie folgt: Im Labor wird das Volumen der aus den Fallen gelassenen Proben notiert. Falls eine Phasentrennung zu beobachten ist, wird eine geringe Menge Ethylenglykolmonomethylether zur Probe gegeben, die Probe geschüttelt und nochmals das Volumen bestimmt. Anschließend werden in einem Szintillations-Vial pipettiert:

| | |
|---|---|
| 3ml | Probenvolumen (Ethanolamin/Diethylenglykolmonobutylether bzw. Ethylenmonomethylether) |
| 5 ml | deionisiertes H₂O |
| 12 ml | Hionic-Fluor |

Anschließend wird die Probe im Szintillationszähler gemessen.

Die PU-Schwämme werden am Soxhlet extrahiert, die Extrakte vereinigt und am Rotationsverdampfer eingegeengt. Die eingeengte Probe wird mit 5 ml Aceton wieder aufgenommen und in kleine Erlenmeyerkolben umgefüllt. Mit der Meßpipette wird beim Umfüllen das Probevolumens bestimmt und entsprechend dokumentiert. Zur Szintillationsmessung kommen

| | | |
|---|---|---|
| | 1 ml | Probe |
| + | 15 ml | Ultima Gold XR |

in einem Kunststoff- oder Glas-Vial in einem Szintillationszähler.

Falls sich in der Pflanzenkammer Kondenswasser bildet, wird dieses über den angebrachten Glashahn am unteren Ende der Pflanzenkammer abgelassen und auf Radioaktivität untersucht. Dazu wird das gesamte Volumen des Kondenswassers bestimmt und anschließend davon 10 ml in 10 ml Ultima Gold XR zur Messung im Szintillationszähler gebracht.

Im folgenden soll der Einsatz des erfindungsgemäßen Systems an Hand eines Versuchsbeispiels erläutert werden.

### Analyse des Verhaltens des Herbizids Isoproturon

Das Herbizid Isoproturon wurde auf die Oberfläche des erfindungsgemäßen Lysimeters appliziert und verschiedene Versuchsparameter mittels der erfindungsgemäßen Lysimeteranlage untersucht. Die Daten zeigen die Ergebnisse von vier Lysimetern, die jeweils mit verschiedenen Böden gefüllt waren.

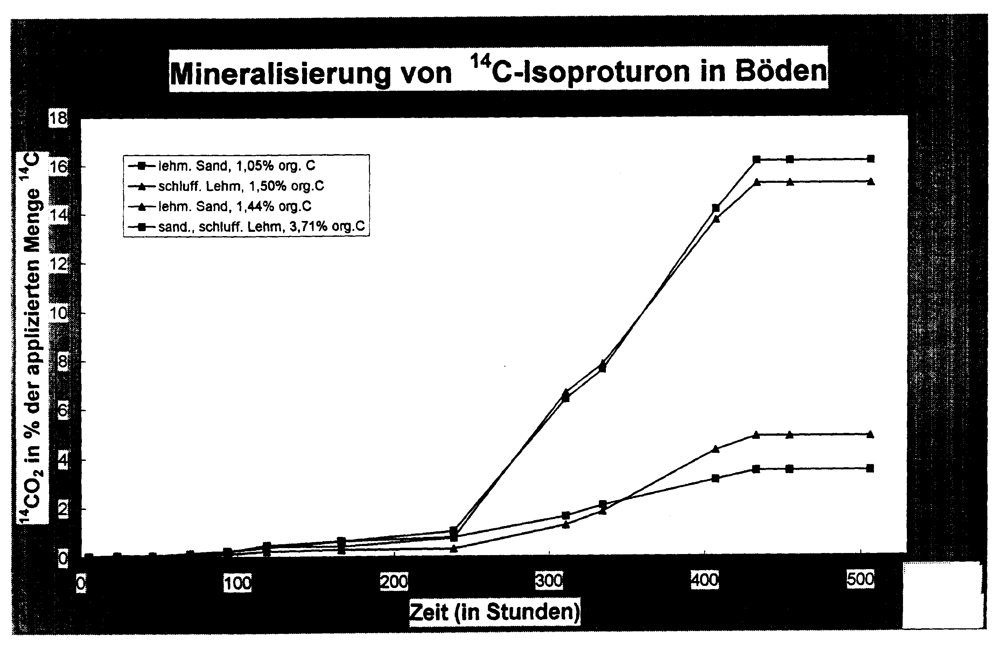

Abbildung 2 zeigt die Mineralisierung von ¹⁴C-Isoproturon in Böden.

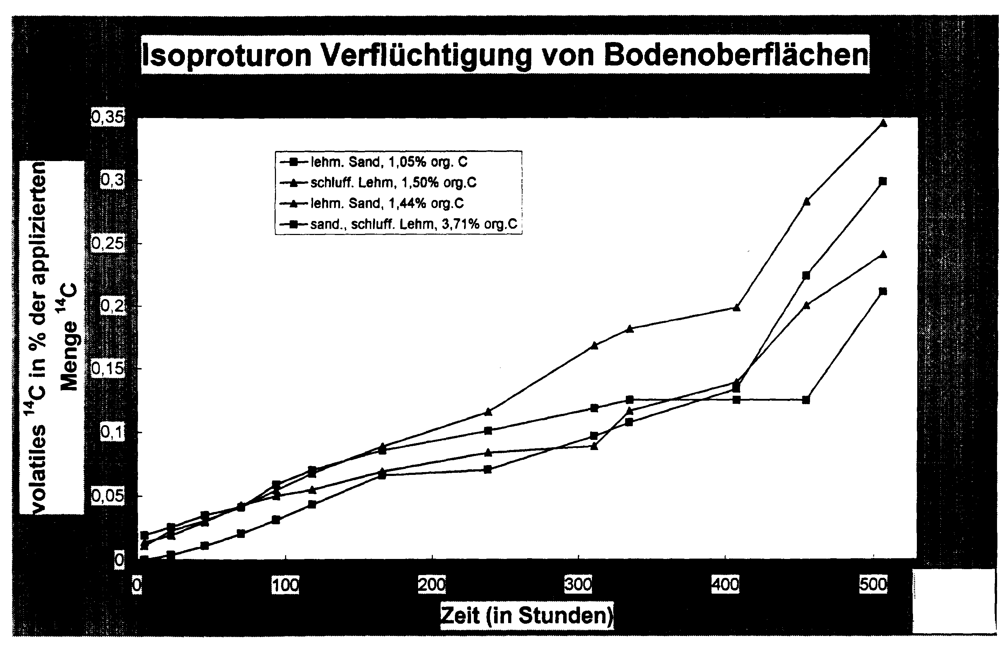

Abbildung 3 zeigt die Verflüchtigung von Isoproturon von den Bodenoberflächen.

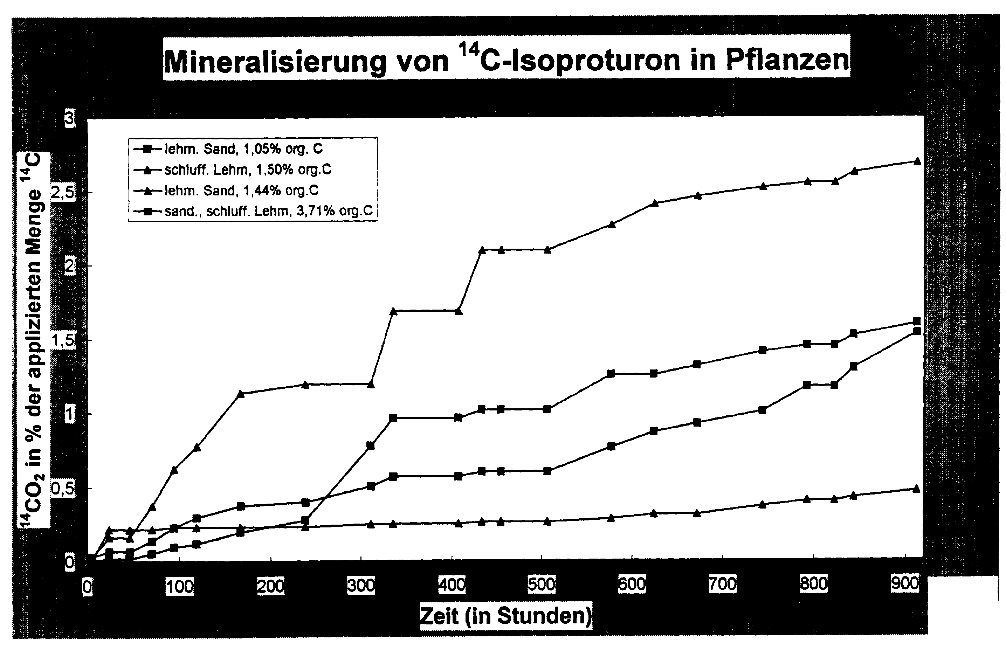

Abbildung 4 zeigt die Mineralisierung von ¹⁴C-Isoproturon in Pflanzen.

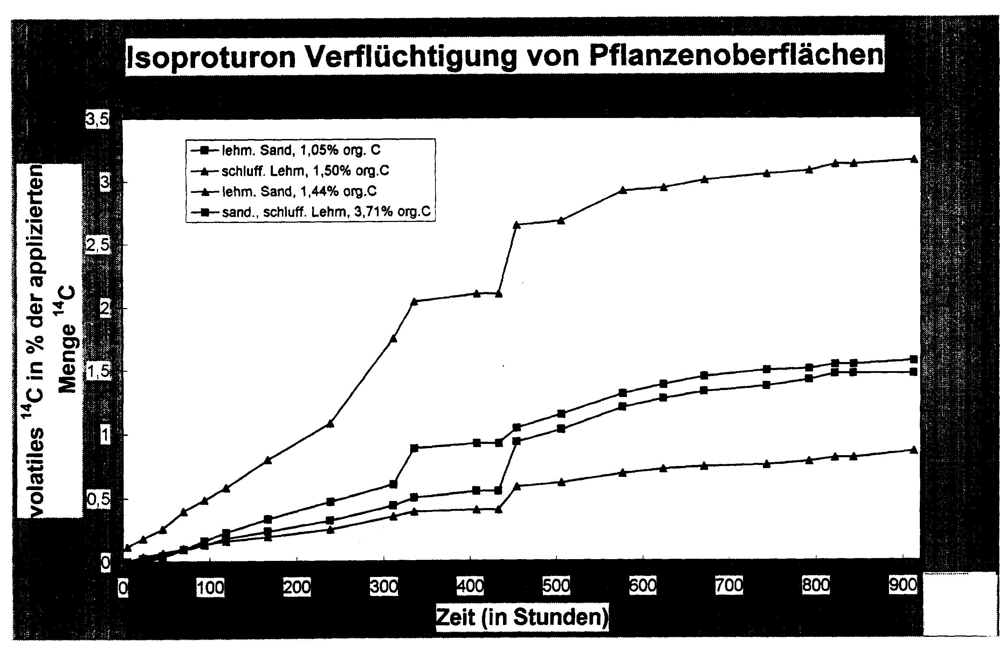

Abbildung 5 zeigt die Verflüchtigung von Isoproturon von Pflanzenoberflächen.

Im Unterschied zu den Lysimeteranlagen des Standes der Technik kann hier eindeutig zwischen den Einzelprozessen Volatilisierung und Mineralisierung von Pflanzenoberflächen einerseits und Volatilisierung und Mineralisierung von Bodenoberflächen andererseits unterschieden werden.

## Patentansprüche

1. Lysimeteranlage zur Untersuchung des Verhaltens von Umweltchemikalien, insbesondere Pflanzenschutzmitteln, umfassend mindestens ein im wesentlichen mit Boden gefülltes Behältnis und eine oberhalb der Bodenoberfläche angeordnete Volatilitätskammer, **dadurch gekennzeichnet,** daß die Volatitätskammer die flüchtigen Verbindungen aus einem definierten Bodenteilbereich erfaßt.

2. Lysimeteranlage nach Anspruch 1, zusätzlich umfassend eine Pflanzenkammer, die die flüchtigen Verbindungen von Pflanzenoberflächen erfaßt.

3. Lysimeteranlage nach Anspruch 1 oder 2, die mindestens eine mit einer Kühlzone ausgestattete Intensivwaschflasche zur Auswaschung der aus der Volatitätskammer und/oder der aus der Pflanzenkammer gelangenen Luft umfaßt.

4. Verfahren zur Untersuchung des Verhaltens von Umweltchemikalien, insbesondere Pflanzenschutzmitteln, bei dem eine Lysimeteranlage nach einem der vorangehenden Ansprüche eingesetzt wird.

5. Verfahren nach Anspruch 4, bei dem die Umweltchemikalien radioaktiv markiert, insbesondere ¹⁴C-markiert, sind.
